Europäisches Patentamt

**(19)** European Patent Office

Office européen des brevets

**(11)** Publication number : **0 443 413 B1**

## EUROPEAN PATENT SPECIFICATION

**(45)** Date of publication of patent specification :
13.04.94 Bulletin 94/15

**(51)** Int. Cl.$^5$: **A61K 31/20, A61K 7/48**

**(21)** Application number : **91101930.5**

**(22)** Date of filing : **12.02.91**

**(54)** Pharmaceutical composition having depigmentating and antiphotoaging activity.

**(30)** Priority : **20.02.90 IT 1942290**

**(43)** Date of publication of application :
**28.08.91 Bulletin 91/35**

**(45)** Publication of the grant of the patent :
**13.04.94 Bulletin 94/15**

**(84)** Designated Contracting States :
**AT BE CH DE DK ES FR GB GR IT LI LU NL SE**

**(56)** References cited :
**EP-A- 0 308 919**
**WO-A-88/00465**
**US-A- 4 877 805**

**(73)** Proprietor : **Schering Spa**
**Via L. Mancinelli 7/11**
**I-20131 Milano (IT)**
Proprietor : **CILAG S.P.A.**
**Via Michelangelo Buonarroti, 23**
**I-20093 Cologno Monzese (Milano) (IT)**

**(72)** Inventor : **Caputo, Ruggero**
**Via Mauro Macchi 65**
**I-20100 Milano (IT)**

**(74)** Representative : **Minoja, Fabrizio**
**Studio Consulenza Brevettuale Via Rossini, 8**
**I-20122 Milano (IT)**

Jouve, 18, rue Saint-Denis, 75001 PARIS

EP 0 443 413 B1

## Description

The present invention relates to dermatologic preparations containing trans-retinoic acid (tretinoin) and azelaic acid as active ingredients.

Recent studies documented the depigmentating action of both trans-retinoic acid and azelaic acid; this property is utilized for the preparation of compositions effective for the treatment of some unaesthetic forms of skin hyperchromatism (melanosis), well visible especially on face and hands.

It has now been found that the trans-retinoic acid and azelaic acid combination exhibits a depigmentating action analogous to the one obtained utilizing the two active ingredients separately but that the results are already evident after three months of therapy. On the contrary, with the two substances used separately, a treatment of at least six months is necessary to observe the desired effect.

Trans-retinoic acid (U.S. Patent 4,603,146, EP-A-0230498) and, to a less extent, azelaic acid (EP-A-0336880), exercise a preventive and reparative action of tissues which run into morphological alterations ascribable to physiological ageing and to damages caused by chronic exposure to actinic radiation.

The antiageing and antiphotoaging actions of retinoic acid take place through the control of mechanisms that govern the regulation of the normal cell replication at the skin level, and in parallel, stimulating the slowed function of structures present in the dermis (fibroblasts, microcirculation, etc.). The clinical expression of these tissural alterations consists in the appearance of wrinkledness (of variable intensity), anomalous pigmentations, focal hyperkeratosis, folliculate ostia marked evidence, skin compactness and tonicity modifications, etc.

The combination of trans-retinoic and azelaic acids proved to be therapeutically more effective than the separate active ingredients in exercising a control and reparative action towards skin structures altered by ageing and photoexposure. Therefore, the pharmaceutical compositions of the present invention can be used by subjects of any age for whom the treatment of skin modifications (visible and non visible) that respond well to trans-retinoic and azelaic acids and to their combination is suitable.

The compositions object of the present invention, which are suitable for topical administration, can be prepared resorting to well-known techniques and excipients, such as, for instance, those described in "Remington's Pharmaceutical Sciences Handbook", Mack Pub.Co. N.Y. USA.

Examples of suitable formulations comprise creams, ointments, gels, lotions, emulsions and the like.

The weight-percentage of trans-retinoic acid can range from 0.005% to 0.05%, preferably from 0.01% to 0.025%, compared to the total amount of the formulation, while the azelaic acid amount can range from 10% to 30%, preferably from 15% to 25%.

The administration form and the treatment length will usually be determined by a physician, but, generally, 1 or 2 applications a day, for a period from 1 to 6 months, will be sufficient to obtain clinically evident results.

Hereinafter, some examples of formulations are reported in a table form; the results obtained in a clinical experimentation compared with the active ingredients not combined each other are reported as well.

## EXAMPLES OF COMPOSITIONS (CREAMS) ACCORDING TO THE

## INVENTION

|  | EX. 1 | EX. 2 | EX. 3 |
|---|---|---|---|
| AZELAIC ACID | 20.00 | 20.00 | 20.00 |
| RETINOIC ACID | 0.01 | 0.02 | 0.05 |
| BUTYLHYDROXY-TOLUENE | 0.10 | 0.10 | 0.10 |
| POLYOXYL 40 STEARATE | 2.30 | 2.30 | 2.30 |
| STEARYL ALCOHOL | 1.38 | 1.38 | 1.38 |
| STEARIC ACID | 8.73 | 8.73 | 8.73 |
| ISOPROPYL MYRISTATE | 4.60 | 4.60 | 4.60 |
| XANTHAN GUM | 0.30 | 0.30 | 0.30 |
| SORBIC ACID | 0.20 | 0.20 | 0.20 |
| DISTILLED WATER | 62.38 | 62.37 | 62.34 |

A product in the form of a cream containing 20% azelaic acid and retinoic acid in two different concentrations (0.01% and 0.025%) was tested on 120 people to evaluate antiphotoageing effect and depigmentating activity.

The evaluation of antiphotoaging effect was performed on 90 patients (two of them were male) while the evaluation depigmentating activity was carried out on 30 female patients suffering from face melanosis.

The patients were photographed after the 1st, 3rd, and 6th months of the treatment. Latero-orbital region skin moulds with silicone material were performed on about 50% of the patients under antiphotoaging treatment at time 0 and after 6 months. Cutaneous moulds were examined with the computer profilometric analysis technique. During the experimentation 18 dropouts occurred (due to personal and not to tolerability reasons). Possible problems of tolerability to the product (burning sensation, slight colour) occurred only during the first days of the treatment and were easily overcome by alternating the evening application of the product with a one day rest.

DEPIGMENTATING ACTIVITY

The product depigmentating activity was evidenced already after the 3rd month in about 1/3 of the treated cases, and it was comparable to that obtained in former experiments with azelaic acid or retinoic acid alone, after a much longer period of treatment (6 months or longer).

ANTIPHOTOAGEING ACTIVITY

The antiphotoageing activity of the product was evaluated not only clinically (by photography) but also by sophisticated profilometric analysis of cutaneous moulds and compared to analogous observations carried out on moulds of patients treated with azelaic acid or retinoic acid only. The results are hereinafter reported.

Instrumental evaluation of the antiphotoageing effect of the azelaic and retinoic acid combination by topical use.

PROFILOMETRIC ANALYSIS

In the group of patients treated with the association the analysis was performed on 15 moulds, selected among the available 52 ones.

17 moulds of subjects of the same age, subjected to the topical treatment with 0.05% retinoic acid and 12 moulds of subjects treated with 20% azelaic acid were analyzed by comparison for the same period.

Summarizing:

a) 20% azelaic acid = 12 patients

b) 0.05% retinoic acid = 17 patients

c) retinoic-azelaic acid combination = 15 patients

To perform the profilometric study, silicone hard resin counter-moulds were prepared before and after the treatment, on the same skin portion, considering two main wrinkles as the reference point. The following parameters were considered: 3 for wrinkledness (RA, RZD, RT), 2 for peak density (NR, D) and one for profile length (Lo). The results obtained for the various parameters can be summarized as following:

**RT parameter**

Maximum wrinkledness.

The distance between the lines tangent to the peaks and to the grooves, within the length of the measured profile.

```
AZ = azelaic acid,        Basal = 40.5    Final = 39.9

Not significant result.

RA = retinoic acid,       Basal = 33.4,   Final = 30.4

Not significant result.


AR+AZ = retinoic + azelaic acid

                          Basal = 45.7    Final = 36.8

Highly significant result.
```

**RZD parameter**

Mean deepness

The mean value of the filtered profile width, measured within five adjacent bases lengths.

```
AZ = azelaic acid         Basal = 26.2    Final = 25

Not significant result.

RA = retinoic Acid        Basal = 21.9    Final = 19.4

Significant result.

RA+AZ=retinoic + azelaic acid

                          Basal = 30.7    Final = 24.8

Highly significant result.
```

**RA parameter**

Wrinkledness mean value.

Mean value of all the ordinates of the filtered profile (from form and corrugation errors), in absolute magnitude, within the length of measure).

4

```
AZ = azelaic acid          Basal = 4.2     Final = 4.1
Not significant result.
RA = retinoic Acid         Basal = 3.5     Final = 3
Not significant result.
RA+AZ = retinoic + azelaic acid
                           Basal = 4.67    Final = 3.5
Highly significant result.
```

After the treatment, skin microwrinkledness is reduced in a statistically significative manner by using the retinoic acid and azelaic acid combination.

Also by using trans-retinoic acid or azelaic acid alone for a six months period a wrinkledness reduction was obtained for the considered parameters but it was statistically no significative.

**NR parameter.**

Peaks number
The peaks number was counted with the density criterion D but referred to 10 mm of profile.

```
AZ = azelaic acid          Basal = 328.8  Final = 375.9
Not significant result.
RA = retinoic acid         Basal = 123.1  Final = 181
Significant result.
RA+AZ = retinoic + azelaic acid
                           Basal = 1131.3 Final = 1602.9
Highly significant result.
```

**D parameter**

Density
Filter and profile peaks (or grooves) number, gathered within a set out band delimitated by the secants parallel to the mean line and named $c_1$ (upper) and $c_2$ (lower) and related to the measured length.

```
AZ = azelaic acid          Basal = 68.8    Final = 78.2
Significant result.
RA = retinoic acid         Basal = 29.1    Final = 42.1
Not significant result.
RA+AZ = retinoic + azelaic acid
                           Basal = 236.1   Final = 328
Highly significant result.
```

**LO Parameter**

Profile development
Filtered profile development length.


```
AZ = azelaic acid          Basal = 1.221  Final = 1.232

Not significant result.



RA = retinoic Acid         Basal = 1.108  Final = 1.118

Not significant result.

RA+AZ = retinoic +  azelaic acid

                           Basal = 1.674  Final = 1.732

Not significant result.
```


From the above data it is evident that the application of the trans-retinoic and azelaic acid combination induced an increase in peak density; this means a return to the "juvenile" type skin texture.

Azelaic and trans-retinoic acids alone showed no better activity in increasing peak density parameters as compared to their association.

The results from the clinical and laboratory researches performed to demonstrate the efficacy of the cream containing the 20% azelaic acid and 0.01% to 0.025% trans-retinoic acid combination allow to express the following observations:

1) the enhanced depigmentating activity of the combination is nearly superimposable to the one achieved with products based only on azelaic acid which were formerly tested (about 35% of regression of the lesions), but the depigmentating activity occurs faster with the combination (about 3 months against 6).

2) Remarkable antiaging activity is unquestionably reported from profilometric analysis of moulds which enables to put in evidence how the azelaic and trans-retinoic acids combination induces a lowering of skin microwrinkledness higher than the one achieved with azelaic or trans-retinoic acid alone.

In conclusion, the combination warrants fast and satisfying depigmentating activity on face melanosis and proves to be particularly efficient in reducing the skin microwrinkledness, one of the most significant clinical expressions of skin damage originated from photoexposure and from ageing processes.


**Claims**

**Claims for the following Contracting States : DE, GB, FR, IT, NL, SE, LI, CH, BE, AT, LU, DK, ES, GR**

1. Dermatologic compositions based on an azelaic and trans-retinoic (tretinoin) acid combination.

2. Dermatologic compositions according to claim 1, in which azelaic acid and trans-retinoic acid are present in concentrations ranging from 10% to 30% and from 0.005% to 0.05% (w/w) respectively.

3. Dermatologic compositions according to claim 1, in which azelaic acid and trans-retinoic acid are present in 20% and 0.01% (w/w) concentrations respectively.

4. Dermatologic compositions according to claim 1, in which azelaic acid and trans-retinoic acid are present in 20% and 0.025% (w/w) concentrations respectively.

5. Dermatologic compositions according to the preceding claims, under the form of day-cream, night-cream, lotions, serum, gels, masks.

6. The use of a azelaic and trans-retinoic acid combination to prepare a dermatologic composition for antiphotoaging and depigmentating skin treatment.

**Patentansprüche**

**Patentansprüche für folgende Vertragsstaaten : DE, GB, FR, IT, NL, SE, LI, CH, BE, AT, LU, DK, ES, GR**

1. Dermatologische Zusammensetzungen basierend auf einer Azelain- und trans-Retinoin-(Tretinoin)-säurekombination.

2. Dermatologische Zusammensetzungen nach Anspruch 1, worin Azelainsäure und trans-Retinoinsäure in Konzentrationen, die von 10 % bis 30 % bzw. von 0,005 % bis 0,05 % (Gew./Gew.) reichen, vorliegen.

3. Dermatologische Zusammensetzungen nach Anspruch 1, worin die Azelainsäure und trans-Retinoinsäure in Konzentrationen von 20 % bzw. 0,01 % (Gew./Gew.) vorliegen.

4. Dermatologische Zusammensetzungen nach Anspruch 1, worin Azelainsäure und trans-Retinoinsäure in Konzentrationen von 20 % bzw. 0,025 % (Gew./Gew.) vorliegen.

5. Dermatologische Zusammensetzungen nach den vorangehenden Ansprüchen, in Form von Tagescreme, Nachtcreme, Lotionen, Serum, Gelen, Masken.

6. Verwendung einer Azelain- und trans-Retinoinsäurezusammensetzung zur Herstellung einer dermatologischen Zusammensetzung zur antiphotoalternden und depigmentierenden Hautbehandlung.

**Revendications**

**Revendications pour les Etats contractants suivants : DE, GB, FR, IT, NL, SE, LI, CH, BE, AT, LU, DK, ES, GR**

1. Compositions dermatologiques basées sur une combinaison d'acide azélaique et d'acide transrétinoique (trétinoine).

2. Compositions dermatologiques selon la revendication 1, dans lesquelles l'acide azélaique et l'acide transrétinoique sont présents en concentrations comprises respectivement entre 10 % et 30 % et 0,005 % et 0,05 % (p/p).

3. Compositions dermatologiques selon la revendication 1, dans lesquelles l'acide azélaique et l'acide transrétinoique sont présents respectivement aux concentrations de 20 % et 0,01 % (p/p).

4. Compositions dermatologiques selon la revendication 1, dans lesquelles l'acide azélaique et l'acide transrétinoique sont présents respectivement aux concentrations de 20 % et 0,025 % (p/p).

5. Compositions dermatologiques selon les revendications précédentes, sous forme de crème de jour, de crème de nuit, de lotions, de sérum, de gels, de masques.

6. Utilisation d'une combinaison d'acide azélaique et d'acide transrétinoique pour préparer une composition dermatologique pour traitement antiphotovieillissement et de dépigmentation de la peau.